# FASCICULE DE BREVET EUROPEEN

(11) **EP 2 897 600 B1**
(45) Date de publication et mention de la délivrance du brevet: **31.01.2018**
(21) Numéro de dépôt: 13774756.4
(22) Date de dépôt: 18.09.2013
(51) Int. Cl.: A61K 31/135, A61K 31/197, A61K 31/343, A61P 21/00

(54) **TRAITEMENT DES NEURONOPATHIES MOTRICES**
BEHANDLUNG VON MOTORISCHEN NEUROPATHIEN
TREATMENT OF MOTOR NEURONOPATHIES

(30) Priorité: 19.09.2012 FR 1258796
(43) Date de publication de la demande: 29.07.2015
(73) Titulaire: Centre National de la Recherche Scientifique (C.N.R.S.), 75794 Paris Cedex 16 (FR)
(72) Inventeur: LEFEBVRE, Suzie, F-75205 Paris Cedex 13 (FR); KHOOBARRY, Kevinee, F-75205 Paris Cedex 13 (FR); BURLET, Philippe, F-75015 Paris (FR)
(74) Mandataire: Cabinet Plasseraud
(86) Numéro de dépôt international: PCT/FR2013/052157
(87) Numéro de publication internationale: WO 2014/044972

(56) Documents cités:
- WO-A1-00/41688
- WO-A2-2006/086544
- WO-A2-2011/156481
- CN-A- 102 274 157
- WAGATSUMA A ET AL: "Effect of treatment with nifedipine, an L-type calcium channel blocker, on muscular atrophy induced by hindlimb immobilization", SCANDINAVIAN JOURNAL OF MEDICINE AND SCIENCE IN SPORTS, vol. 12, no. 1, février 2002 (2002-02), pages 26-30, XP002695383, ISSN: 0905-7188
- HUGON JACQUES: "ALS therapy: Targets for the future", NEUROLOGY, vol. 47, no. 6 SUPPL. 4, 1996, pages S251-S254, XP002695384, ISSN: 0028-3878
- SZCZUDLIK A ET AL: "[Assessment of the efficacy of treatment with pimozide in patients with amyotrophic lateral sclerosis. Introductory notes].", NEUROLOGIA I NEUROCHIRURGIA POLSKA 1998 JUL-SEP, vol. 32, no. 4, juillet 1998 (1998-07), pages 821-829, XP009168684, ISSN: 0028-3843
- MILLER R G ET AL: "A clinical trial of verapamil in amyotrophic lateral sclerosis", MUSCLE AND NERVE, vol. 19, no. 4, 1996, pages 511-515, XP002695385, ISSN: 0148-639X
- MILLER R G ET AL: "Controlled trial of nimodipine in amyotrophic lateral sclerosis", NEUROMUSCULAR DISORDERS 199603 GB, vol. 6, no. 2, mars 1996 (1996-03), pages 101-104, XP002695386, ISSN: 0960-8966

## Description

L'invention concerne un antagoniste calcique choisi dans le groupe des antagonistes calciques de la famille des phénylalkylamines pour son utilisation dans le traitement d'une neuronopathie motrice.

L'amyotrophie spinale infantile (ou SMA, *Spinal Muscular Atrophy*) est une maladie neuromusculaire à transmission autosomique récessive. Elle est caractérisée par une dégénérescence des motoneurones alpha de la corne antérieure de la moelle épinière entraînant une atrophie musculaire et conduisant à la paralysie. Cette dégénérescence des neurones moteurs alpha compromet donc sensiblement le pronostic vital des patients. Chez le sujet sain, ces neurones transmettent les messages du cerveau aux muscles, entraînant la contraction de ces derniers. En l'absence d'une telle stimulation, les muscles s'atrophient. Par la suite, outre une faiblesse et une atrophie généralisées des muscles, et plus particulièrement de ceux du tronc, du haut des bras et des cuisses, ces troubles peuvent s'accompagner de graves problèmes respiratoires.

Il existe une forte corrélation entre l'âge d'apparition des symptômes et la sévérité de l'atteinte, de sorte que, plus la maladie commence précocement et moins le pronostic vital est favorable. C'est selon ce critère que cette maladie a été classée en 3 types cliniques comme suit:
- le type I (maladie de Wernig-Hoffman), caractérisé par un début précoce, généralement avant 6 mois et dans lequel la station assise n'est jamais acquise et l'évolution gravissime. L'espérance de vie des enfants atteints dépasse rarement trois ans et se limite souvent à quelques mois.
- le type II ou forme intermédiaire de la maladie de Wernig-Hoffman qui apparaît habituellement entre l'âge de six mois et de trois ans, dans lequel la station assise est acquise, mais la marche ne l'est jamais. Ce type clinique est souvent associé à de fréquentes infections respiratoires pouvant compliquer la cause de cette affection et diminuer l'espérance de vie.
- le type III ou maladie de Kugelberg-Welander, qui apparaît généralement vers 3-4 ans et parfois jusqu'à 21 ans et dans lequel la marche est acquise avec des troubles de la démarche plus ou moins prononcés suivant la sévérité qui est très variable d'un patient à l'autre. Ce type de la maladie en général ne compromet pas l'espérance de vie.

Toutes les formes d'amyotrophie spinale s'accompagnent d'une faiblesse et d'une atrophie progressive des muscles, par suite de la dégénérescence des neurones de la corne antérieure de la moelle épinière. La SMA constitue actuellement l'une des causes de mortalité infantile la plus fréquente. Elle touche indifféremment filles ou garçons dans toutes les régions du monde avec une prévalence comprise entre 1/6000 et 1/10000.

Le gène impliqué dans les amyotrophies infantiles spinales a été localisé sur le chromosome 5 en position q12-q13, quel que soit le type clinique présenté. Ce gène code pour la protéine de survie des motoneurones (SMN). Cette protéine est localisée dans le cytoplasme et le noyau, où elle s'accumule dans des domaines ponctiformes appelés Corps de Cajal ou Cajal bodies (CBs). Un défaut de cette accumulation est décrit dans la SMA.

Il est admis que la SMA est liée à une inactivation du gène SMN. Plus précisément, il a été mis en lumière deux gènes codant la protéine SMN : SMN1 et SMN2, les 2 gènes étant transcrits. L'analyse de leurs promoteurs a montré que ces éléments sont quasiment identiques tant au niveau de leur séquence qu'au niveau de leur activité. Aussi, le gène SMN2 code pour la même protéine SMN mais en moindre quantité. Il a d'ailleurs été constaté que l'inactivation du SMN1 peut être palliée en partie par l'expression du gène SMN2 quasi-identique.

Les patients atteints de SMA disposent actuellement de soins médicaux ou paramédicaux leur assurant la meilleure qualité de vie possible, tels que:
- la kinésithérapie motrice et l'hydrothérapie qui consistent à aider le patient à constituer son schéma corporel;
- la kinésithérapie respiratoire qui permet de soulager les patients de l'encombrement des bronches, diminuant ainsi le risque d'affections respiratoires; ou encore
- la prise en charge orthopédique pour prévenir les déformations du squelette et des articulations.

Toutefois, ces soins ne visent qu'à soulager des symptômes et prévenir les complications de la maladie. Ils ne permettent pas de soigner la pathologie.

D'autre part, la sclérose latérale amyotrophique (SLA) ou maladie de Charcot est une maladie neurodégénérative touchant principalement des adultes, et caractérisée par un affaiblissement puis une paralysie des muscles des jambes et des bras, des muscles respiratoires, ainsi que des muscles de la déglutition et de la parole. La prévalence de cette maladie est estimée à 1 malade sur 25 000 personnes.

Cette maladie implique également la dégénérescence des neurones moteurs, en particulier ceux du cortex cérébral et de la corne antérieure de la moelle épinière. Cette dégénérescence conduit à une destruction du faisceau pyramidal. La SLA peut se présenter sous deux formes principales : la forme « spinale » et la forme « bulbaire ». La forme spinale est due à la dégénérescence des motoneurones situés dans la moelle épinière tandis que la forme bulbaire correspond à la dégénérescence des motoneurones du bulbe rachidien. Ces deux formes peuvent se succéder ou se développer simultanément, la maladie progressant presque toujours vers une forme complète (spinale et bulbaire).

Les patients souffrant de SLA disposent de soins médicaux visant non pas à la récupération des fonctions motrices mais à l'entretien des fonctions restantes, notamment grâce à la rééducation kinésithérapeute et orthophonique. En outre, il a été récemment proposé une stratégie thérapeutique se basant sur le Riluzole (commercialisé sous la dénomination Rilutek®). Mais cette stratégie permet uniquement de retarder l'évolution de la pathologie efficace et prolonge la phase de la maladie durant laquelle le patient est autonome. Aussi, ce composé améliore le pronostic des patients de seulement 30 %, ce qui reste insatisfaisant. Il n'existe actuellement aucun traitement efficace contre l'apparition et/ou le développement de la SLA.

Il n'existe aucun traitement en vue de stopper de manière efficace la progression des neuronopathies motrices, en particulier pour freiner la dégénérescence des neurones moteurs. Il existe donc un besoin de longue date pour une stratégie thérapeutique efficace contre des neuronopathies motrices, en particulier les neuronopathies motrices de type amyotrophie spinale infantile et sclérose latérale amyotrophique.
Wagatsuma A et al., Scandinavian Journal of Medicine and Science in Sport, 2002, décrit l'effet bénéfique de la nifédipine sur l'atrophie musculaire.
Hugon J, Neurology, 1996, divulgue le potentiel de l'utilisation des antagonistes calciques dans le traitement de la sclérose latérale amyotrophique.
Szczudlik A, Neurologia, 1998, décrit un ralentissement de la progression de la sclérose latérale amyotrophique grâce à l'administration de l'antagoniste calcique pimozide.
Miller et al, Muscle and Nerve, 1996, décrit l'intérêt du verapamil dans le traitement de la sclérose latérale amyotrophique.Miller et al, Neuromuscular Disorders, 1996, décrit l'intérêt du nimodipine dans le traitement de la sclérose latérale amyotrophique.
La demande de brevet CN 102 274 157 divulgue une composition comprenant de la flunarizine et de la nicergoline.
La demande WO2006086544 décrit des compositions comprenant des antagonistes de la VEGF et des antagonistes calciques tels que la flunarizine.
La demande WO2011156481 décrit des compositions comprenant de l'amiodarone et une cyclodextrine substituée.
La demande WO00/41688 décrit l'utilisation de l'amiodarone comme agent antifongique.

Les inventeurs ont maintenant découvert que des antagonistes calciques de la famille des phénylalkylamines, des acides aminés et des benzofuranes permettent d'une part d'augmenter de manière significative la distribution de SMN dans les CBs et d'autre part, d'améliorer un défaut d'accumulation des snRNPs dans les CBs. Par conséquent, ces inhibiteurs calciques constituent une stratégie thérapeutique pertinente dans le traitement des neuronopathies motrices, en particulier la SMA ou la SLA.

Il est décrit ici un antagoniste calcique choisi dans le groupe constitué :
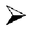 des antagonistes calciques de la famille des phénylalkylamines de formule I et leurs sels, 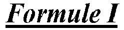 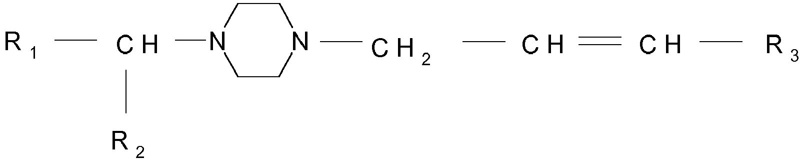 dans laquelle :
   - les groupements R₁, R₂ et R₃ sont identiques ou différents et sont choisis parmi les radicaux phényles et fluorophényles, pourvu qu'au moins un des groupements R₁, R₂ et R₃ soit un radical fluorophényle;
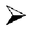 des antagonistes calciques de la famille des acides aminés de formule II,

   ***Formule II*** H₂N-CH₂-C(R₄)R'₄-CH₂-COOR₅

   dans laquelle :
   - le groupement R₄ est un radical alkyl inférieur à chaine linéaire ou ramifiée contenant jusqu'à 8 atomes de carbone et le groupement R'₄ est un atome d'hydrogène,
      *ou*
      R₄ et R'₄ forment ensemble un radical alkyl inférieur cyclique contenant jusqu'à 8 atomes de carbone, et
   - le groupement R₅ est un atome d'hydrogène ou un radical alkyl inférieur à chaine linéaire ou ramifiée contenant jusqu'à 8 atomes de carbone;
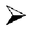 des antagonistes calciques de la famille des benzofuranes de formule III et leurs sels, 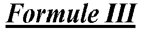 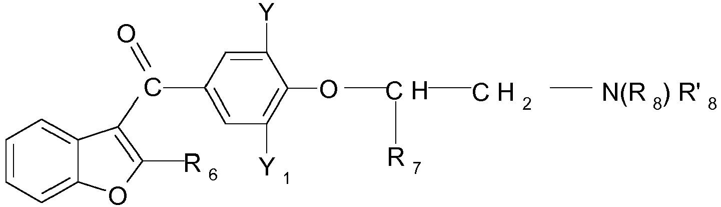 dans laquelle :
   - le groupement R₆ représente un radical alkyl inférieur à chaine linéaire ou ramifiée contenant jusqu'à 6 atomes de carbone,
   - le groupement R₇ représente un atome d'hydrogène ou un groupement méthyl,
   - le groupement N(R₈)R'₈ représente un groupement diméthylamino, diéthylamino, dipropylamino, pipéridino, pyrrolidino ou morpholino, et
   - les groupements Y et Y₁ sont identiques et représentent un atome d'hydrogène, d'iode ou de brome;
   pour son utilisation dans le traitement d'une neuronopathie motrice choisie parmi l'amyotrophie spinale infantile, la sclérose latérale amyotrophique, la sclérose latérale primaire, la maladie de Kennedy, et la maladie de Charcot-Marie-Tooth.

L'invention concerne un antagoniste calcique choisi dans le groupe constitué des antagonistes calciques de la famille des phénylalkylamines de formule I et leurs sels, 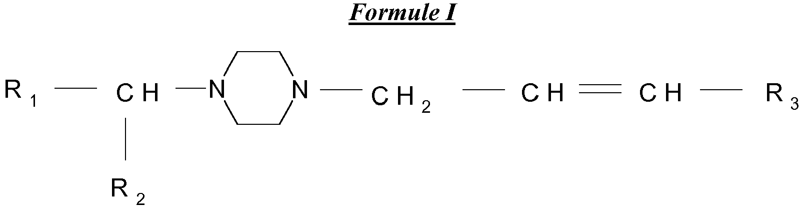 dans laquelle :
- les groupements R₁, R₂ et R₃ sont identiques ou différents et sont choisis parmi les radicaux phényles et fluorophényles, pourvu qu'au moins un des groupements R₁, R₂ et R₃ soit un radical fluorophényle;
pour son utilisation dans le traitement d'une neuronopathie motrice choisie parmi l'amyotrophie spinale infantile, la sclérose latérale amyotrophique, la sclérose latérale primaire, la maladie de Kennedy et la maladie de Charcot-Marie-Tooth.

Par "**neurones moteurs**" ou "**motoneurones**", on entend les neurones constituant la voie de sortie du système nerveux central (ou la voie finale) de tout acte moteur. Les corps cellulaires des motoneurones sont situés soit dans le tronc cérébral, soit dans la corne ventrale de la substance grise de la moelle épinière. Chaque motoneurone possède un axone qui part du système nerveux central pour innerver les fibres musculaires d'un muscle. L'ensemble constitué par un motoneurone et les fibres musculaires qu'il innerve constitue une unité motrice. On distingue trois types de motoneurones: les "**motoneurones alpha**", qui innervent les fibres musculaires responsables de la contraction, les "**motoneurones gamma**", qui innervent les fuseaux neuromusculaires, ajustant ainsi leur sensibilité à l'étirement, ainsi que les **"motoneurones beta"**, qui innervent les deux types de fibres. Préférentiellement, le composé de l'invention est particulièrement avantageux pour le traitement de neuronopathies motrices impliquant la dégénérescence des neurones moteurs alpha.

Par "**neuronopathie motrice**", on entend une maladie impliquant la dégénérescence des neurones moteurs, qui se manifeste par une absence de stimulation des muscles conduisant à une amyotrophie.
Cette maladie est associée à un déficit en protéine SMN et/ou à une réduction du nombre de CBs et/ou à un défaut de localisation de la protéine SMN aux CBs.
Les symptômes d'une telle pathologie peuvent être variés et peuvent comprendre:
- un déficit moteur progressif;
- une amyotrophie;
- des fasciculations; et/ou
- des crampes.

Ladite neuronopathie motrice est l'amyotrophie spinale infantile, la sclérose latérale amyotrophique, la sclérose latérale primaire, la maladie de Kennedy, ou la maladie de Charcot-Marie-Tooth. Préférentiellement, ladite neuronopathie motrice est l'amyotrophie spinale infantile, la sclérose latérale amyotrophique. Plus préférentiellement, ladite neuronopathie est l'amyotrophie spinale infantile. Typiquement, ladite amyotrophie spinale infantile est de type I, de type II ou de type III.

Par "**antagoniste calcique"** ou "**inhibiteur calcique"** on entend un composé qui inhibe l'entrée du calcium dans les cellules par les canaux calciques voltage-dépendants. Cette classe thérapeutique est particulièrement bien documentée dans l'art antérieur, en particulier pour leur utilisation en cardiologie, notamment dans le traitement d'hypertension artérielle, l'angor ou encore des crises de tachycardie jonctionnelle paroxystique.
Ces antagonistes calciques sont décrits, entre autres, par Théophile Godfraind dans le manuel Calcium Channel Blockers, publié par les éditions Milestones in Drug Therapy le 26 mars 2004.

Les inventeurs ont maintenant mis en lumière un nouveau rôle de ces antagonistes calciques. En effet, ils ont montré que ces derniers permettent l'augmentation de la distribution de la protéine SMN et des snRNPs aux Corps de Cajal et/ou l'augmentation du nombre de Corps de Cajal.
Aussi, les antagonistes calciques de l'invention, en augmentant de manière significative la distribution de la protéine SMN et des snRNPs aux Corps de Cajal et/ou le nombre de Corps de Cajal, constitue une stratégie thérapeutique pertinente pour le traitement des neuronopathies motrices, en particulier la SMA.

Par "**Corps de Cajal"** ou **"Cajal bodies**" ou encore "CBs" on entend le site des modifications initiales et de l'assemblage de plusieurs petits ARNs nucléaires avec leurs ribonucléoprotéines, les snRNPs (small nuclear ribonucleoproteins) importés depuis le cytoplasme, ou recyclé dans le noyau. Les CBs présentent une ultra structure révélant des fils enroulés, d'où son autre nom de « coiled bodies ». Les CBs sont des structures dynamiques et leur nombre peut varier rapidement s'il y a un changement du niveau de transcription. L'abondance des CBs peut être aisément déterminée par l'homme du métier, en particulier par l'immuno-détection de la localisation des snRNPs aux CBs et le nombre de CBs ainsi détecté, ou plus précisément par la détection de la protéine coiline aux CBs. Comme indiqué précédemment, la protéine SMN s'accumule dans les Corps de Cajal. Un défaut de cette accumulation est d'ailleurs décrit dans la SMA.

Les inventeurs ont également mis en évidence le fait que les antagonistes calciques de l'invention permettent l'augmentation des snRNPs au niveau des Corps de Cajal.

La protéine SMN fait partie d'un large complexe multiprotéique, dont le rôle est l'assemblage de particules ribonucléoprotéiques de l'épissage, les snRNPs. Un défaut de cet assemblage est visualisé par une diminution marquée de la localisation des snRNPs au niveau des CBs dans les fibroblastes de patients SMA. Aussi, les antagonistes calciques de l'invention, en augmentant cette accumulation de snRNPs au niveau des CBs permettent de traiter, avec efficacité, les neuronopathies motrices, en particulier la SMA.

### Antagoniste calcique de la famille des phénylalkylamines

L'antagoniste calcique de invention est un antagoniste de la famille des phénylalkylamines de formule I ou l'un de leurs sels: 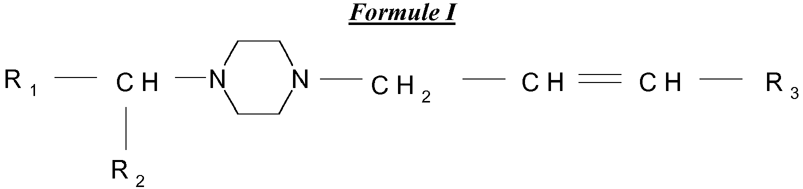 dans laquelle :
- les groupements R₁, R₂ et R₃ sont identiques ou différents et sont choisis parmi les radicaux phényles et fluorophényles, pourvu qu'au moins un des groupements R₁, R₂ et R₃ soit un radical fluorophényle.

Cette classe d'antagonistes calciques est décrite par Théophile Godfraind dans le manuel Calcium Channel Blockers, publié par les éditions Milestones in Drug Therapy le 26 mars 2004. Ces antagonistes calciques de formule I sont également décrits dans la demande FR 2 014 487 (Janssen Pharmaceutica). La formule I de l'invention englobe tous les stéréoisomères géométriques Z ou E. Préférentiellement, la formule I de l'invention est sous la configuration E.

Dans un mode de réalisation préféré:
- les groupements R₁ et R₂ sont des radicaux parafluorophényles; et
- le groupement R₃ est un radical phényl.
Dans ce mode de réalisation, ledit antagoniste calcique de la famille des phénylalkylamines est la flunarizine ou l'un de ses sels. De manière préférée, il s'agit du dichlorhydrate de flunarizine. Aussi, l'invention se rapporte à la flunarizine ou l'un de ses sels pour son utilisation dans le traitement d'une neuronopathie motrice choisie parmi l'amyotrophie spinale infantile, la sclérose latérale amyotrophique, la sclérose latérale primaire, la maladie de Kennedy et la maladie de Charcot-Marie-Tooth.
En effet, les inventeurs ont montré que ce composé présente les meilleurs résultats quant à l'augmentation de l'accumulation de la protéine SMN et/ou des snRNPs dans les CBs. Typiquement, le dichlorhydrate de flunarizine peut être administré à une dose comprise entre 5 mg et 10 mg par jour.

La flunarizine a pour formule C₂₆H₂₈Cl₂F₂N₂ telle que représentée en formule Ia:

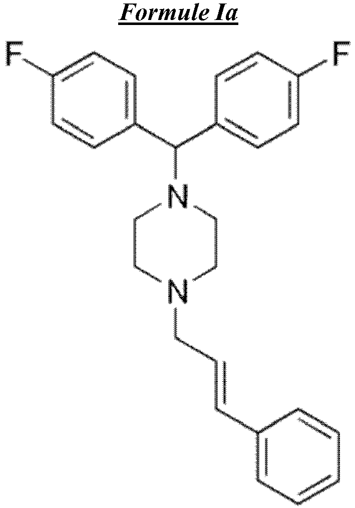

La flunarizine porte le numéro CAS: 30484-77-6. Cette molécule est connue pour son application dans le traitement des migraines et est commercialisée sous le nom SIBELIUM®. Il s'agit d'un inhibiteur des canaux calciques des neurones et des canaux calciques des vaisseaux, utilisé dans le traitement des vertiges d'origine vestibulaire et de la migraine.

### Antagonistes calciques de la famille des acides aminés

D'autres antagonistes calciques décrits ici comme composés de référence sont des antagonistes calciques de la famille des acides aminés de formule II:

***Formule II*** H₂N-CH₂-C(R₄)R'₄-CH₂-COOR₅

dans laquelle :
- le groupement R₄ est un radical alkyl inférieur à chaine linéaire ou ramifiée contenant jusqu'à 8 atomes de carbone et le groupement R'₄ est un atome d'hydrogène,
   *ou*
   R₄ et R'₄ forment ensemble un radical alkyl inférieur cyclique contenant jusqu'à 8 atomes de carbone, et
- le groupement R₅ est un atome d'hydrogène ou un radical alkyl inférieur à chaine linéaire ou ramifiée contenant jusqu'à 8 atomes de carbone. Typiquement, les radicaux alkyles inférieurs R₄ ou R₅ de formule II sont des radicaux alkyles de 1 à 4 atomes de carbone, notamment un radical méthyl, éthyl, isopropyle, isobutyle ou tert-butyle.
Cette classe d'antagonistes calciques est décrite par Théophile Godfraind dans le manuel Calcium Channel Blockers, publié par les éditions Milestones in Drug Therapy le 26 mars 2004. Ces antagonistes calciques de formule (II) sont également décrits dans la demande FR 2 294 697 (Warner Lambert Company).
Dans un mode de réalisation préféré:
- R₄ et R'₄ forment ensemble un groupe cylohexane, et
- le groupement R₅ est un atome d'hydrogène.
Dans ce mode de réalisation préféré, ledit antagoniste calcique de la famille des acides aminés est la gabapentine. Aussi, il est décrit ici une gabapentine pour son utilisation dans le traitement d'une neuronopathie motrice choisie parmi l'amyotrophie spinale infantile, la sclérose latérale amyotrophique, la sclérose latérale primaire, la maladie de Kennedy et la maladie de Charcot-Marie-Tooth.

La gabapentine ou acide 2-[1-(aminométhyl)cyclohexyl]acétique a pour formule brute C₉Hₗ₇NO₂ et est représentée par la formule (IIa).

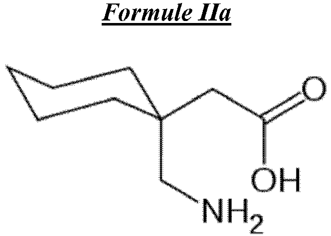

La gabapentine porte le numéro CAS : 60142-96-3. Elle est connue pour son activité antiépileptique. Il est par ailleurs commercialisé sous le nom Neurontin®.

Dans un autre mode de réalisation,
- le groupement R₄ est un isobutyle, et
- les groupements R'₄ et R₅ sont un atome d'hydrogène.
Dans ce mode de réalisation particulier, ledit antagoniste calcique de la famille des acides aminés est la prégabaline. Aussi, il est décrit une prégabaline pour son utilisation dans le traitement d'une neuronopathie motrice choisie parmi l'amyotrophie spinale infantile, la sclérose latérale amyotrophique, la sclérose latérale primaire, la maladie de Kennedy et la maladie de Charcot-Marie-Tooth.

La prégabaline a pour formule brute C₈H₁₇NO₂ et est représentée en formule IIb :

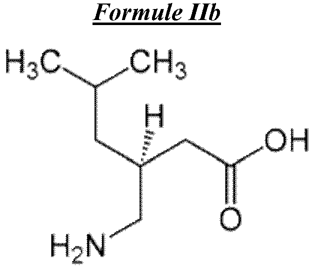

La prégabaline porte le numéro CAS 148553-50-8. Elle est connue pour son activité antiépileptique.

### Antagonistes calciques de la famille des benzofuranes

Dans un autre mode de réalisation, les composés de formule I peuvent être administrés en association avec un antagoniste de la famille des benzofuranes de formule III ou l'un de ses sels: 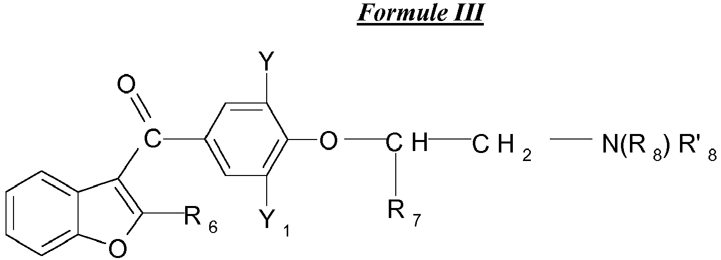 dans laquelle:
- le groupement R₆ représente un radical alkyl inférieur à chaine linéaire ou ramifiée contenant jusqu'à 6 atomes de carbone,
- le groupement R₇ représente un atome d'hydrogène ou un groupement méthyl,
- le groupement N(R₈)R'₈ représente un groupement diméthylamino, diéthylamino, dipropylamino, pipéridino, pyrrolidino ou morpholino, et
- les groupements Y et Y₁ sont identiques et représentent un atome d'hydrogène, d'iode ou de brome.

Cette classe d'antagonistes calciques est décrite par Théophile Godfraind dans le manuel Calcium Channel Blockers, publié par les éditions Milestones in Drug Therapy le 26 mars 2004. Ces antagonistes calciques de formule (III) sont également décrits dans la demande FR 1 339 389 (Société belge de l'azote et des produits chimiques du Marly).

Dans un mode de réalisation préféré,
- le groupement R₆ est un butyle,
- le groupement R₇ est un atome d'hydrogène,
- le groupement N(R₈)R'₈ est un groupement diéthylamino, et
- les groupements Y et Y₁ sont identiques et sont un atome d'iode.
Dans ce mode de réalisation, l'antagoniste de la famille des benzofuranes est l'amiodarone ou l'un de ses sels. Aussi, il est décrit une amiodarone ou l'un de ses sels pour son utilisation dans le traitement d'une neuronopathie motrice choisie parmi l'amyotrophie spinale infantile, la sclérose latérale amyotrophique, la sclérose latérale primaire, la maladie de Kennedy et la maladie de Charcot-Marie-Tooth.

L'amiodarone ou le (2-butyl-3-benzofuranyl)[4-[2-(diéthylamino)éthoxy]-3,5-diiodophényl]méthanone chlorhydrate est un composé de formule brute C₂₅H₂₉I₂NO₃ et est représenté par la formule IIIa.

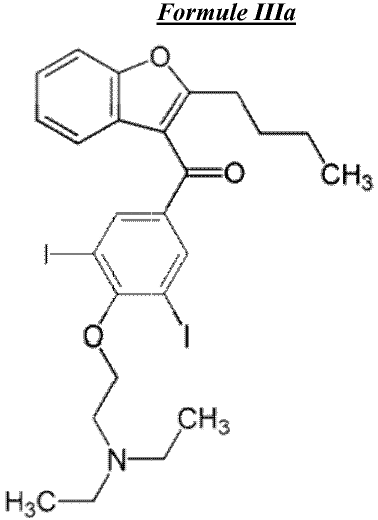

L'amiodarone porte le numéro CAS : 96027-74-6. Il est connu pour son activité antiarythmique.

### Méthode de traitement

Il est également décrit les antagonistes calciques de formule I pour l'utilisation dans des méthodes de traitement d'un sujet souffrant d'une neuronopathie motrice, telle que l'amyotrophie spinale infantile, la sclérose latérale amyotrophique, la sclérose latérale primaire, la maladie de Kennedy ou encore la maladie de Charcot-Marie-Tooth comprenant l'étape d'administration audit sujet une quantité thérapeutiquement efficace d'au moins les antagonistes calciques de l'invention. Par "quantité thérapeutiquement efficace" on entend une quantité suffisante pour traiter et/ou traiter ou stopper la progression de neuronopathies motrices.

### Compositions pharmaceutiques

Les antagonistes calciques de l'invention peuvent également être utilisés pour préparer des compositions pharmaceutiques pour le traitement de neuronopathies motrices telles que l'amyotrophie spinale infantile, la sclérose latérale amyotrophique, la sclérose latérale primaire, la maladie de Kennedy ou la maladie de Charcot-Marie-Tooth. Ainsi, l'invention concerne des compositions pharmaceutiques pour une utilisation dans des méthodes de traitement de neuronopathies motrices chez l'homme, lesdites compositions comprenant au moins un antagoniste calcique selon l'invention et un véhicule pharmaceutiquement acceptable.

Les antagonistes calciques de l'invention sont connus et sont disponibles sur le marché. On pourra utiliser les formulations déjà développées et employées pour chacun des antagonistes calciques de l'invention. N'importe quel antagoniste calcique selon l'invention peut être combiné à tout type de véhicule ou excipient pharmaceutiquement acceptable, en particulier ceux déjà décrits dans l'art antérieur pour leur utilisation pour les antagonistes calciques de l'invention, pour former une composition pharmaceutique selon l'invention. Le terme "pharmaceutiquement acceptable" se réfère à des entités moléculaires et des compositions qui ne produisent pas de réaction inverse, allergique ou autre réaction non désirée lorsqu'elles sont administrées à un mammifère, particulièrement un humain. Un véhicule ou excipient pharmaceutiquement acceptable peut être solide, semi-solide ou liquide.

La forme des compositions pharmaceutiques, leur voie d'administration, leur dosage et leur posologie dépendent naturellement de la sévérité de la pathologie, de son stade d'évolution, de l'âge, du sexe, du poids du sujet à traiter, etc. L'homme du métier veillera donc à adapter les dosages en fonction du patient à traiter, en particulier lors du traitement de la SMA dans laquelle les patients sont des enfants.

Les compositions pharmaceutiques selon l'invention peuvent être formulées pour une administration topique, orale, systémique, intra-nasale, parentérale, intraveineuse, intramusculaire, sous-cutanée, ou autre. Selon le mode d'administration, la composition comprenant un antagoniste calcique selon l'invention peut se présenter sous toutes les formes galéniques.

De préférence, l'antagoniste calcique selon l'invention est compris dans une composition qui est administrée par voie orale. Par voie orale, les compositions peuvent se présenter sous forme de comprimés, de gélules, de dragées, de sirops, de suspensions, de solutions, de poudres, de granulés, d'émulsions, de microsphères ou nanosphères ou vésicules lipidiques ou polymériques permettant une libération contrôlée.

Pour une application topique sur la peau, la composition peut avoir la forme notamment de solution aqueuse ou huileuse ou de dispersion du type lotion ou sérum ; d'émulsion de consistance liquide ou semi-liquide du type lait, obtenue par dispersion d'une phase grasse dans une phase aqueuse (H/E) ou inversement (E/H) ; d'émulsion de consistance molle du type crème ; d'émulsion biphasée; de gel aqueux ou anhydre ; de mousse ou encore de microcapsules ou microparticules, ou de dispersions vésiculaires de type ionique et/ou non ionique, ou encore de formules pulvérisables de type «spray». Ces compositions sont préparées selon les méthodes usuelles connues de l'homme de l'art.

Pour une application par voie systémique, la composition peut se présenter sous forme de solution aqueuse ou huileuse ou sous forme de sérum.

Dans un mode de réalisation préféré, la composition selon l'invention comprend un antagoniste calcique de la famille des phénylalkylamines selon l'invention et un antagoniste calcique de la famille des benzofuranes selon l'invention.
Aussi, l'invention concerne également une composition comprenant :
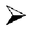 un antagoniste calcique de la famille des phénylalkylamines de formule I et ses sels, 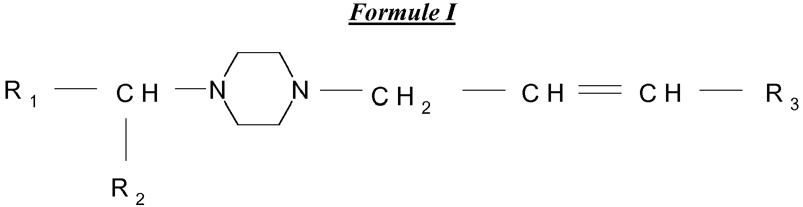 dans laquelle :
   - les groupements R₁, R₂ et R₃ sont identiques ou différents et sont choisis parmi les radicaux phényles et fluorophényles, pourvu qu'au moins un des groupements R₁, R₂ et R₃ soit un radical fluorophényle;
   et
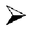 un antagoniste calcique de la famille des benzofuranes de formule III et ses sels 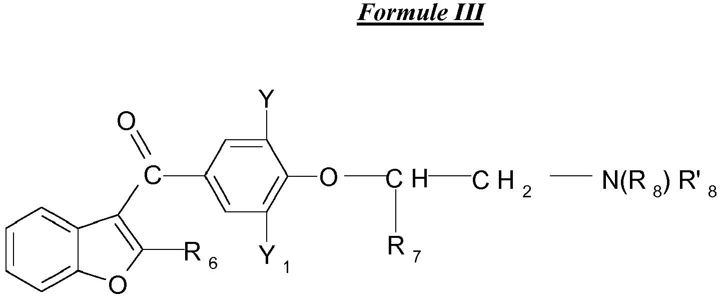 dans laquelle :
   - le groupement R₆ représente un radical alkyl inférieur à chaine linéaire ou ramifiée contenant jusqu'à 6 atomes de carbone,
   - le groupement R₇ représente un atome d'hydrogène ou un groupement méthyl,
   - le groupement N(R₈)R'₈ représente un groupement diméthylamino, diéthylamino, dipropylamino, pipéridino, pyrrolidino ou morpholino, et
   - les groupements Y et Y₁ sont identiques et représentent un atome d'hydrogène, d'iode ou de brome;
   pour son utilisation dans le traitement d'une neuronopathie motrice choisie parmi l'amyotrophie spinale infantile, la sclérose latérale amyotrophique, la sclérose latérale primaire, la maladie de Kennedy et la maladie de Charcot-Marie-Tooth.

Toutes les caractéristiques techniques citées précédemment s'appliquent ici.

Préférentiellement, ledit antagoniste calcique de la famille des phénylalkylamines de formule I est la flunarizine, et plus préférentiellement le dichlorhydrate de flunarizine. Préférentiellement, ledit antagoniste calcique de la famille des benzofuranes de formule III est l'hydrochloride d'amiodarone.

Les inventeurs ont en effet montré que l'administration combinée du dichlorhydrate de flunarizine et de l'hydrochloride d'amiodarone exerce un effet de synergie, allant au delà de la simple accumulation des effets desdits deux composés pris séparément.

Aussi, le dichlorhydrate de flunarizine et l'hydrochloride d'amiodarone agissent en synergie sur la distribution de la protéine SMN aux CBs chez les fibroblastes de patients.

Par conséquent, l'invention concerne également une composition comprenant du dichlorhydrate de flunarizine et de l'hydrochloride d'amiodarone pour son utilisation dans le traitement d'une neuronopathie motrice choisie parmi l'amyotrophie spinale infantile, la sclérose latérale amyotrophique, la sclérose latérale primaire, la maladie de Kennedy et la maladie de Charcot-Marie-Tooth.

Dans ce mode de réalisation particulier, la combinaison de dichlorhydrate de flunarizine et l'hydrochloride d'amiodarone est administrée à une dose inférieure à 5 mg par jour. En réduisant sensiblement la dose de principe actif administrée pour traiter un patient souffrant d'une neuronopathie motrice, les inventeurs ont mis au point une stratégie thérapeutique qui réduit considérablement les risques d'apparition d'effets néfastes e/ou secondaires liés au traitement. Une telle stratégie thérapeutique s'avère donc particulièrement pertinente, en particulier dans le traitement chronique de patients en bas âge souffrant de neuronopathie motrice.

La composition selon l'invention peut associer les antagonistes calciques selon l'invention à d'autres agents actifs, notamment des agents actifs susceptibles de traiter les neuronopathies motrices. Parmi ces agents actifs, on peut citer à titre d'exemple:
- le riluzole, (Haddad H et al. Riluzole attenuates spinal muscular atrophy disease progression in a mouse model, 2003. 28(4):432-437; et Wadman RI et al.. 2012. Drug treatment for spinal muscular atrophy type I, Cochrane Database Syst Rev. 4:CD006281);
- l'hydroxyurée (Chen TH et al, Randomized, double-blind, placebo-controlled trial of hydroxyurea in spinal muscular atrophy, Neurology, 2010 75:2190-21);
- les inhibiteurs de l'ubiquitine/protéasome (Chang HC et al., Degradation of survival motor neuron (SMN) protein is mediated via the ubiquitin/proteasome pathway, Neurochem Int., 2004, 45:1107-1112; et Kwon DY et al., Increasing expression and decreasing degradation of SMN ameliorate the spinal muscular atrophy phenotype in mice, Hum Mol Genet., 2011, 20:3667-3677);
- les inhibiteurs des histone déacétylases (Lunke S et al., The emerging role of epigenetic modifications and chromatin remodeling in spinal muscular atrophy. J Neurochem, 2009, 109(6):1557-1569);
- les inhibiteurs des protéines phosphatases (Novoyatleva T et al., Protein phosphatase 1 binds to the RNA recognition motif of several splicing factors and regulates alternative pre-mRNA processing, Hum. Mol. Genet., 2008, 17, 52-70);
- l'olésoxime (TRO19622), Bordet T et al., Identification and characterization of cholest-4-en-3-one, oxime (TRO19622), a novel drug candidate for amyotrophic lateral sclerosis, J Pharmacol Exp Ther, 2007, 322(2):709-720.);
- les aminoglycosides (Mattis VB et al., Analysis of a read-through promoting compound in a severe mouse model of spinal muscular atrophy, 2012, 525(1):72-75);
- le salbutamol (Pane M et al., Daily salbutamol in young patients with SMA type II, Neuromuscul Disord., 2008, 18:536-540);
- l'isoindoline (demande US 2010/0267712);
- l'ibudilast (demande US 2009/0062330);
- les modulateurs des kinases (Burnett BG, et al., Regulation of SMN protein stability, Mol Cell Biol., 2009, 29(5):1107-1115);
- les variants d'IGF-1 (Murdocca M et al., IPLEX administration improves motor neuron survival and ameliorates motor functions in a severe mouse model of SMA, Mol Med. 2012 May 29. doi: 10.2119/molmed.2012.00056);
- les facteurs de croissance humains (demande US 2008/0187512);
- les pyrimidines bicycliques inhibant la PTK (Hastings ML et al., Tetracyclines that promote SMN2 exon 7 splicing as therapeutics for spinal muscular atrophy, Sci Transl Med., 2009, 1(5):5ra12);
- la quinazoline inhibant l'EGFR (Butchbach ME et al., Effects of 2,4-diaminoquinazoline dérivatives on SMN expression and phenotype in a mouse model for spinal muscular atrophy, Hum Mol Genet., 2010, 19(3):454-467);
- les inhibiteurs de tyrosine kinases (demande US 2010/0305036);
- les inhibiteurs de VEGFR (demande US 2010/0331296);
- les inhibiteurs de l'HSP90 (Suzuki K et al., Pathogenesis-targeting therapeutics for spinal and bulbar muscular atrophy. (SBMA), Neuropathology, 2009, 29(4):509-516);
- les antagonistes de la myostatine (Rose FF Jr et al., Delivery of recombinant follistatin lessens disease severity in a mouse model of spinal muscular atrophy, Hum Mol Genet., 2009, 18(6):997-1005);
- l'héparine à faible poids moléculaire (demande US 2002/0040013);
- la néramexane (demande US 2010/0234402);
- la nicergoline (demande US 2003/0134869);
- les inhibiteurs de la protéine kinase G3SKB (Makhortova NRet al., A screen for regulators of survival of motor neuron protein levels, Nat Chem Biol., 2011, 7(8):544-552);
- les inhibiteurs de RhoA et de Rho-associated kinase (ROCK pathway) (Bowerman M et al., Mol Cell Neurosci, 2009, 42:66-74 et Bowerman M et al., Hum Mol Genet 2010, 19: 1468-1478);
- -les antioxydants (Wan L et al., Inactivation of the SMN complex by oxidative stress, Mol Cell., 2008 31(2):244-254);
- les inhibiteurs de l'apoptose (Sareen D et al., Inhibition of apoptosis blocks human motor neuron cell death in a stem cell model of spinal muscular atrophy. PLoS One. 7(6):e39113. 2012); ou encore
- les modulateurs des voies ERK et PI3K/AKT kinases. (Biondi O et al.. In vivo NMDA receptor activation accelerates motor unit maturation, protects spinal motor neurons, and enhances SMN2 gene expression in severe spinal muscular atrophy mice, J Neurosci., 2010 30:11288-11299.)

Aussi, il est également décrit ici une composition comprenant au moins un antagoniste calcique choisi dans le groupe constitué :
- des antagonistes calciques de la famille des phénylalkylamines de formule I et leurs sels, 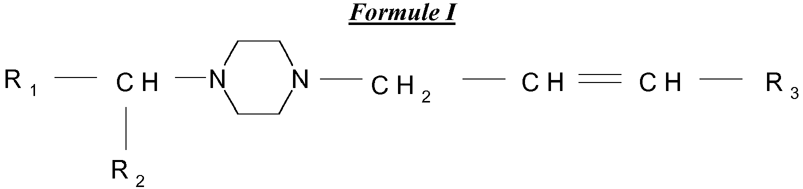 dans laquelle :
   - les groupements R₁, R₂ et R₃ sont identiques ou différents et sont choisis parmi les radicaux phényles et fluorophényles, pourvu qu'au moins un des groupements R₁, R₂ et R₃ soit un radical fluorophényle;
- des antagonistes calciques de la famille des benzofuranes de formule III et leurs sels 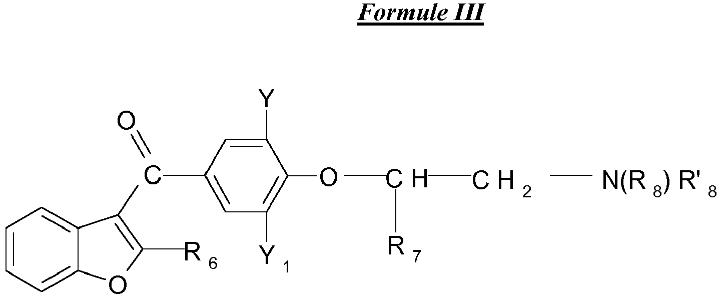 dans laquelle :
   - le groupement R₆ représente un radical alkyl inférieur à chaine linéaire ou ramifiée contenant jusqu'à 6 atomes de carbone,
   - le groupement R₇ représente un atome d'hydrogène ou un groupement méthyl,
   - le groupement N(R₈)R'₈ représente un groupement diméthylamino, diéthylamino, dipropylamino, pipéridino, pyrrolidino ou morpholino, et
   - les groupements Y et Y₁ sont identiques et représentent un atome d'hydrogène, d'iode ou de brome;
et
au moins un agent actif choisi parmi le riluzole, l'hydroxyurée, les inhibiteurs de l'ubiquitine/protéasome, les inhibiteurs des histone déacétylases, les inhibiteurs des protéines phosphatases, l'olésoxime, les aminoglycosides, le salbutamol, l'isoindoline, l'ibudilast, les modulateurs des kinases, les variants d'IGF-1, les facteurs de croissance humain, les pyrimidines bicycliques inhibant la PTK, la quinazoline inhibant l'EGFR, les inhibiteurs de tyrosine kinases, les inhibiteurs de l'HSP90, les antagonistes de la myostatine, l'héparine à faible poids moléculaire, la néramexane, les inhibiteurs de la protéine kinase G3SKB, les inhibiteurs de RhoA et de Rho-associated kinase (ROCK pathway), les anti-oxydants, les inhibiteurs de l'apoptose et les modulateurs des voies ERK et PI3K/AKT kinases.

Préférentiellement, ledit antagoniste calcique est la flunarizine ou un de ses sels.

Typiquement, l'association des antagonistes calciques de l'invention et dudit agent actif peut être simultanée, séparée ou étalée dans le temps.

Typiquement, les antagonistes calciques de l'invention peuvent être associés au Riluzole pour son utilisation dans le traitement de la sclérose latérale amyotrophique.

### FIGURES

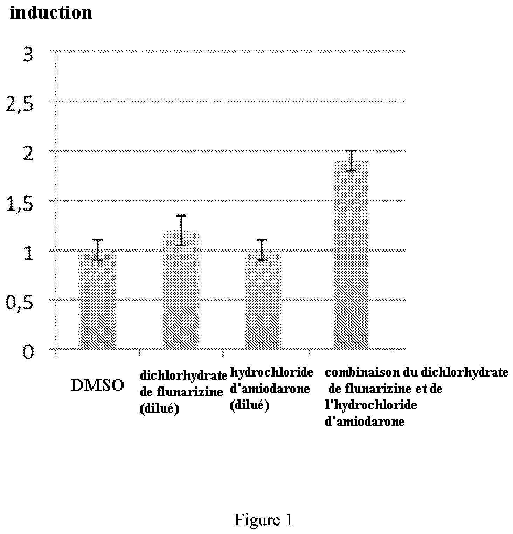

**Figure 1** **:** Effet de synergie entre le dichlorhydrate de flunarizine et l'hydrochloride d'amiodarone sur la distribution de la protéine SMN aux CBs.
Cette figure montre les effets sur les fibroblastes SMA immortalisés :
- du DMSO (témoin pour lequel une valeur d'induction d'un facteur égal à 1 a été arbitrairement attribuée),
- du dichlorhydrate de flunarizine seul,
- de l'hydrochloride d'amiodarone seul, et
- de la combinaison du dichlorhydrate de flunarizine et de l'hydrochloride d'amiodarone.

### EXEMPLES

### 1. Test des antagonistes calciques de l'invention sur les fibroblastes SMA immortalisés

Les inventeurs ont montré un effet bénéfique des antagonistes calciques de la famille des phénylalkylamines, de la famille des acides aminés et ceux de la famille des benzofuranes sur le nombre de Corps de Cajal de fibroblastes SMA immortalisés.
Pour ce faire, ils ont testé des molécules caractéristiques représentant le mieux chacune de ces familles:
- le dichlorhydrate de flunarizine,
- la gabapentine,
- la prégabaline, et
- le chlorhydrate d'amiodarone.

L'immortalisation à l'aide de l'antigène grand T du virus SV4O de fibroblastes isolés à partir d'un enfant atteint d'une forme sévère de la maladie a permis d'établir des conditions de culture cellulaire particulièrement robustes. Cette immortalisation est décrite dans la publication Lefebvre S, Burlet P, Viollet L, Bertrandy S, Huber C, Belser C et Munnich A. A novel association of the SMN protein with major non-ribosomal nucleolar proteins and its implication in spinal muscular atrophy. Hum Mol Genet (2002) 11 (9) : 1017-1027.

L'augmentation du nombre de CBs dans les fibroblastes SMA immortalisés (10000 à 12000 cellules par puits de 0,8 à 1 cm carré dans un milieu de culture contrôlé) a été évaluée après 24 heures de traitement pour chacun des antagonistes calciques à une concentration de 2 microgrammes/ml (i.e. entre 2 et 10 micromolaires, suivant la masse moléculaire de la substance). Les antagonistes de l'invention sont disponibles dans le commerce (par exemple chez Sigma-Aldrich) et sont vendus en plaques de 96 puits, à 2 milligrammes par molécule par puits à dissoudre dans 1 ml de DMSO.

La concentration de 2 microgrammes/ml (soit une dilution 1:1000 de la solution-mère) correspond à une action maximale pour tous les antagonistes. L'analyse par immunomarquage à l'aide d'anticorps dirigés contre la protéine SMN (commerciaux et produits à façon) montre que cette protéine se localise occasionnellement au niveau des CBs dans 3 à 6% des cellules non traitées ou traitées avec le DMSO seul (excipient témoin).

Pour déterminer si les antagonistes calciques exerçaient un effet positif, les inventeurs ont compté les cellules qui avaient la protéine SMN localisée dans les CBs sur 100 cellules choisies au hasard pour chaque expérience.

Cette expérience a été répétée trois fois. Les résultats obtenus à partir de la moyenne des trois premières expériences ont révélé que les molécules testées augmentent de manière significative le nombre de CBs contenant la protéine SMN après 24 heures de traitement à 2 microgrammes/ml (khi-2, p<0.001, n=300, 100 cellules par expérience).

Une valeur d'induction d'un facteur égal à 1 a été est arbitrairement attribuée au DMSO. Les résultats montrent que les 4 molécules exerçaient une induction significative d'un facteur égal à au mois 1,7.

Plus précisément, les inventeurs ont en outre montré que la prégabaline exerce une induction significative d'un facteur égal à 1,7 (khi-2, 0,001<p<0.01, n=2300 cellules). Le chlorhydrate d'amiodarone exerce une induction significative d'environ 2,8. La gabapentine exerce une induction significative d'environ 3,3. Enfin, le chlorhydrate de flunarizine exerce une induction significative d'environ 4,8.

Les inventeurs ont ensuite vérifié que ces molécules exerçaient toujours le même effet bénéfique après 78 heures de traitement avec une seule dose initiale.

L'analyse statistique à partir de ces expériences a permis de conclure que ces molécules étaient capables d'augmenter de manière significative la distribution de la protéine SMN dans les CBs dans la lignée immortalisée de fibroblastes SMA (khi-2, p<0,0001, n>3000 cellules). L'analyse par immunomarquage des protéines SMN et coiline (un autre marqueur des CBs détecté à l'aide d'un anticorps commercial) a confirmé que ces deux protéines étaient bien co-localisées dans les CBs dans les cellules traitées avec chacune des 4 molécules.

Les inventeurs ont ainsi réussi à montrer que ces 4 composés sont particulièrement pertinents dans le traitement de neuronopathies motrices.

### 2. Vérification des effets exercés sur des fibroblastes de patients SMA

La distribution de la protéine SMN aux Corps de Cajal dans une série de culture primaires de fibroblastes de la peau de patients atteints de la forme sévère (type I), intermédiaire (Type II) et modéré de la maladie (type III) induite par l'action des antagonistes calciques de l'invention a été analysée. La valeur moyenne est calculée à partir de 3 à 7 expériences (n=300 à 700 cellules) La valeur de khi-2 est indiqué dans le tableau 1.

Pour confirmer l'effet des antagonistes calciques de l'invention, les inventeurs ont analysé la distribution de la protéine SMN aux Corps de Cajal dans une série de cultures primaires de fibroblastes de la peau de patients atteints de la forme sévère (type I), intermédiaire (Type II) et modérée de la maladie (type III) induite par l'action des antagonistes calciques de l'invention.
Ces fibroblastes sont obtenus chez:
- 2 individus atteints de la forme sévère (type I),
- 2 individus atteints de la forme intermédiaires (type II), et
- 1 individu atteint de la forme modérée (type III).
L'analyse statistique des résultats par le test de khi-2 est résumée dans le tableau 1.

**Tableau 1:Analyste statistique par test de khi-2 de l'action des antagonistes calciques de l'invention sur la distribution de la protéine SMN dans les CBs dans des cultures primaires de fibroblastes de la peau de patients atteints de la forme sévère (type I), intermédiaire (type II), et modérée de la maladie (type III)**

| **Composé** | **Type I** | | **Type II** | | **Type III** |
|---|---|---|---|---|---|
| | **Patient 1** | **Patient 2** | **Patient 3** | **Patient 4** | **Patient 5** |
| **Dichlorhydrate de flunarizine** | 2,87 | 27,44 | 6,09 | 10,99 | 12,38 |
| **Gabapentine** | 5,38 | 36,85 | 3,1 | 0,78 | 5,85 |
| **Chlorhydrate d'amiodarone** | 0,46 | 7,16 | 0 | 1,2 | 2,72 |

L'action des antagonistes calciques de l'invention est plus faible dans les cultures primaires de fibroblastes SMA. Ceci s'explique par un métabolisme cellulaire plus lent des cultures primaires, tandis que la lignée immortalisée se divise plus rapidement.

L'analyse détaillée révèle que les 2 cultures de type I ne réagissent pas de la même manière. L'une des cultures (patient 2) répond positivement aux composés de l'invention sur les cellules immortalisées de type I, alors que la seconde (patient 1) répond de manière significative qu'à la molécule gabapentine (test de khi-2, 0,02<p<0,05). Cette dernière est la lignée GM03813 obtenue chez Coriell Cell Repositories (USA) ; elle est largement utilisée par la communauté scientifique pour mener des études sur l'action de molécules dans le domaine de la SMA.

Les cultures de type II et III répondent globalement de manière plus faible que la culture de type I positive. Cette différence est liée au nombre de CBs avec la protéine SMN au début du traitement, ce nombre étant déjà plus élevé dans les types II et III que dans les types I (Renvoisé B, Khoobarry K, Gendron MC, Cibert C, Viollet L et Lefebvre S. Distinct domains of the spinal muscular atrophy protein SMN are required for targeting to Cajal bodies in mammalian cells. Journal of Cell Sciences (2006) 119: 680-692.

Globalement, cette étude montre que les antagonistes calciques de l'invention sont pertinents pour le traitement de neuronopathies motrices, en particulier la SMA. Toutefois, il apparaît que le dichlorhydrate de flunarizine est le composé qui exerce une action positive pour le plus grand nombre de cultures primaires de patients SMA, les trois types confondus.

Pour évaluer la relation entre la concentration (dose) et l'effet (réponse) sur les CBs des cellules SMA immortalisées, les inventeurs ont construit une courbe dose-réponse pour chacune des molécules. La concentration efficace médiane (EC5O) dans les conditions expérimentales décrites ici est d'environ 80 ± 22 nM pour la dichlorhydrate de flunarizine Les résultats sont répertoriés dans le tableau 2.

**Tableau 2: Action des composés de l'invention sur la distribution de la protéine SMN dans les CBs dans la lignée immortalisée de fibroblastes d'un patient atteint de la forme sévère (type I)**

| **Composé** | **Concentration à 2µg/ml (µM)** | **Maximum d'induction** | **Dilution à EC50** | **EC50, nM** | **Induction à EC50** |
|---|---|---|---|---|---|
| **DMSO** | 1:1000 | 1 ± 0,3 | -- | -- | -- |
| **Dichlorhydrate de flunarizine** | 4,2 | 4,8 ± 1,5 | 1/53 | 80 ± 22 | 2,9 |
| **Gabapentine** | 11,6 | 4,4 ±1,0 | 1/55 | 210 ± 38 | 2,9 |
| **Chlorhydrate d'amiodarone** | 3,0 | 2,8 ± 1,0 | 1/50 | 60 ± 26 | 1,8 |

Plusieurs rapports publiés indiquent que la dose efficace moyenne de dichlorhydrate de flunarizine administrée par voie orale chez l'homme est de 10 mg par jour, ce qui correspond à des valeurs du taux plasmatique maximal de dichlorhydrate de flunarizine d'environ 81 ± 16 nanogrammes/ml (soit d'environ 170 ± 34 nM) dans un temps compris entre 2 et 4 heures et l'état d'équilibre est atteint en 5 à 6 semaines. De plus, la distribution tissulaire est importante et la demi-vie d'élimination est longue. Les concentrations plasmatiques se situent donc dans une fourchette de concentrations compatible avec les concentrations associées aux effets du dichlorhydrate de flunarizine dans notre modèle cellulaire.

La protéine SMN fait partie d'un large complexe multiprotéique, dont le rôle le mieux étudié est l'assemblage de particules ribonucléoprotéiques de l'épissage, les snRNPs (small nucleair ribonucleoproteins). Un défaut de cet assemblage est visualisé par une diminution marquée de la localisation des snRNPs au niveau des CBs dans les fibroblastes de patients SMA (Renvoisé B, Khoobarry K, Gendron MC, Cibert C, Viollet L et Lefebvre S. Distinct domains of the spinal muscular atrophy protein SMN are required for targeting to Cajal bodies in mammalian cells. Journal of Cell Sciences (2006) 119: 680-692). Dans un autre modèle cellulaire, il a été publié que la présence et le nombre de CBs étaient directement liés à l'efficacité avec laquelle les snRNPs sont assemblés (Klingauf M., Stanek, D. and Neugebauer, K.M. (2006). Enhancement of U4/U6 small nuclear ribonucleoprotein particle association in Cajal bodies predicted by mathematical modeling. Mol. Biol. Cell., 2006, 17, 4972-4981.

Des expériences de co-immunomarquage similaires aux précédents ont été menées sur les cellules SMA immortalisées à l'aide de deux anticorps commerciaux, l'un spécifique de la protéine coiline (marqueur des CBs) et l'autre de la coiffe triméthylguanosine (TMG) des petits ARNs nucléaires des snRNPs.

Ces essais montrent une augmentation de la protéine SMN et des snRNPs aux Corps de Cajal dans la lignée immortalisée SMA de la forme sévère de la maladie (Type I) induite par les antagonistes calciques de l'invention. La valeur moyenne pour les snRNPs est calculée à partir des 3 expériences (n=300 cellules). L'analyse de ces immunomarquages montre une augmentation significative des snRNPs au niveau des CBs dans les cellules traitées pendant 24 heures avec le dichlorhydrate de flunarizine (khi-2, 0.001<p< 0.01), à partir de trois expériences indépendantes sur 100 cellules par expérience.

Cette étude montre que le dichlorhydrate de flunarizine est le composé qui exercerait le mieux un effet bénéfique à la fois sur:
- l'accumulation de la protéine SMN dans les CBs; et
- l'accumulation des snRNPs dans les CBs.

En conclusion, cette étude place:
- les antagonistes calciques de la famille des phénylalkylamines, en particulier le flunarizine et ses sels,
- les antagonistes calciques de la famille des acides aminés, et
- les antagonistes calciques de la famille des benzofuranes,
dans une stratégie thérapeutique des maladies impliquant la dégénérescence des neurones moteurs, en particulier l'amyotrophie spinale infantile, la sclérose latérale amyotrophique, la sclérose latérale primaire, la maladie de Kennedy et la maladie de Charcot-Marie-Tooth.

### 3. Effet de synergie entre le dichlorhydrate de flunarizine et l'hydrochloride d'amiodarone sur la distribution de la protéine SMN aux CBs.

Les inventeurs ont testé l'effet de la combinaison du dichlorhydrate de flunarizine et de l'hydrochloride d'amiodarone sur des fibroblastes SMA immortalisés isolés à partir d'un enfant atteint d'une forme sévère de la maladie, conformément aux protocoles décrits aux exemples 1 et 2.

Pour ce faire, ils ont observé l'effet sur les fibroblastes SMA immortalisés :
- du DMSO (témoin pour lequel une valeur d'induction d'un facteur égal à 1 a été arbitrairement attribuée),
- du dichlorhydrate de flunarizine seul,
- de l'hydrochloride d'amiodarone seul, et
- de la combinaison du dichlorhydrate de flunarizine et de l'hydrochloride d'amiodarone.

Notons que le dichlorhydrate de flunarizine et le l'hydrochloride d'amiodarone sont utilisés dans une dilution au 1/1000 par rapport aux exemples 1 et 2.

Les résultats sont représentés en figure 1. La figure 1 montre que le dichlorhydrate de flunarizine et l'hydrochloride d'amiodarone agissent en synergie sur la distribution de la protéine SMN aux CBs.

Plus précisément, les inventeurs ont montré que:
- le dichlorhydrate de flunarizine seul, dilué au 1/1000, exerce une induction significative d'un facteur égal à 1,2 (khi-2, 0,001<p<0.01, n=1072 cellules);
- l'hydrochloride d'amiodarone seul, dilué au 1/1000, exerce une induction non significative d'un facteur égal à 1 (khi-2, 0,001<p<0.01, n=625 cellules);
- le dichlorhydrate de flunarizine et l'hydrochloride d'amiodarone exercent ensemble une induction significative d'un facteur égal à 1,9 (khi-2, p<0.001, n=2625 cellules).

Cet exemple montre l'effet surprenant et avantageux de la combinaison du dichlorhydrate de flunarizine et de l'hydrochloride d'amiodarone. Les inventeurs ont ainsi mis au point une stratégie thérapeutique particulièrement pertinente pour le traitement de neuronopathies motrices.

## Revendications

1. Antagoniste calcique choisi dans le groupe constitué des antagonistes calciques de la famille des phénylalkylamines de formule I et leurs sels, 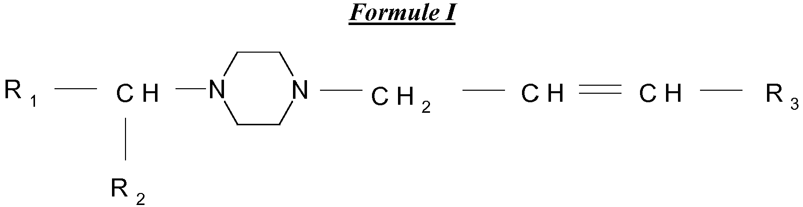 dans laquelle :
- les groupements R₁, R₂ et R₃ sont identiques ou différents et sont choisis parmi les radicaux phényles et fluorophényles, pourvu qu'au moins un des groupements R₁, R₂ et R₃ soit un radical fluorophényle;
pour son utilisation dans le traitement d'une neuronopathie motrice choisie parmi l'amyotrophie spinale infantile, la sclérose latérale amyotrophique, la sclérose latérale primaire, la maladie de Kennedy et la maladie de Charcot-Marie-Tooth.

2. Antagoniste calcique pour son utilisation selon la revendication 1, **caractérisée en ce que** ladite neuronopathie motrice est l'amyotrophie spinale infantile.

3. Antagoniste calcique pour son utilisation selon la revendication 1, **caractérisé en ce que** ledit antagoniste calcique appartient à la famille des phénylalkylamines de formule I ou l'un de leurs sels,
dans laquelle:
- les groupements R₁ et R₂ sont des radicaux parafluorophényles, et
- le groupement R₃ est un radical phényl.

4. Antagoniste calcique choisi dans le groupe constitué de la flunarizine, ses sels, et leurs mélanges pour son utilisation dans le traitement d'une neuronopathie motrice choisie parmi l'amyotrophie spinale infantile, la sclérose latérale amyotrophique, la sclérose latérale primaire, la maladie de Kennedy et la maladie de Charcot-Marie-Tooth.

5. Antagoniste calcique pour son utilisation selon la revendication **4**, ledit antagoniste calcique étant la flunarizine ou l'un de ses sels.

6. Antagoniste calcique pour son utilisation selon la revendication **4**, ledit antagoniste calcique étant le dichlorhydrate de flunarizine.

7. Antagoniste calcique pour son utilisation selon la revendication **6**, **caractérisé en ce que** ledit antagoniste calcique est administré à une dose comprise entre 5 mg et 10 mg par jour.

8. Antagoniste calcique pour son utilisation selon l'une quelconque des revendications **1 à 6**, **caractérisé en ce qu'**il permet l'augmentation de la distribution de la protéine SMN et des snRNPs aux Corps de Cajal et/ou l'augmentation du nombre de Corps de Cajal.

9. Composition comprenant :
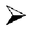 un antagoniste calcique de la famille des phénylalkylamines de formule I et ses sels, 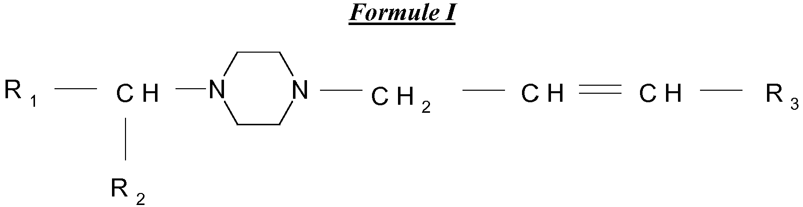 dans laquelle :
- les groupements R₁, R₂ et R₃ sont identiques ou différents et sont choisis parmi les radicaux phényles et fluorophényles, pourvu qu'au moins un des groupements R₁, R₂ et R₃ soit un radical fluorophényle;
et
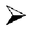 un antagoniste calcique de la famille des benzofuranes de formule III et ses sels 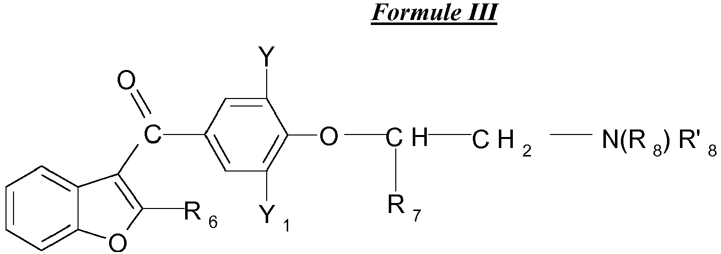 dans laquelle :
- le groupement R₆ représente un radical alkyl inférieur à chaine linéaire ou ramifiée contenant jusqu'à 6 atomes de carbone,
- le groupement R₇ représente un atome d'hydrogène ou un groupement méthyl,
- le groupement N(R₈)R'₈ représente un groupement diméthylamino, diéthylamino, dipropylamino, pipéridino, pyrrolidino ou morpholino, et
- les groupements Y et Y₁ sont identiques et représentent un atome d'hydrogène, d'iode ou de brome;
pour son utilisation dans le traitement d'une neuronopathie motrice choisie parmi l'amyotrophie spinale infantile, la sclérose latérale amyotrophique, la sclérose latérale primaire, la maladie de Kennedy et la maladie de Charcot-Marie-Tooth.

10. Composition pour son utilisation selon la revendication **9**, **caractérisée en ce que** ledit antagoniste calcique de la famille des phénylalkylamines de formule I est le dichlorhydrate de flunarizine et **en ce que** ledit antagoniste calcique de la famille des benzofuranes de formule III est l'hydrochloride d'amiodarone.

11. Composition pour son utilisation selon la revendication **9** ou **10**, **caractérisée en ce qu'**elle est administrée à une dose inférieure à 5 mg par jour.

## Patentansprüche

1. Calciumantagonist, ausgewählt aus der Gruppe bestehend aus Calciumantagonisten der Familie der Phenylalkylamine der Formel I und deren Salze, 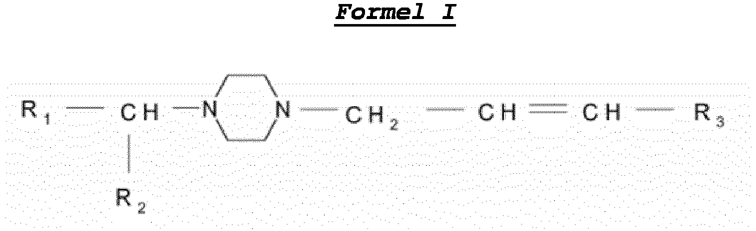 worin:
die Gruppen R₁, R₂ und R₃ identisch oder voneinander verschieden sind und ausgewählt sind aus Phenylresten und Fluorphenylresten, unter der Voraussetzung, dass mindestens eine der Gruppen R₁, R₂ und R₃ ein Fluorphenylrest ist;
zur Verwendung in der Behandlung einer motorischen Neuropathie, ausgewählt aus infantiler spinaler Muskelatrophie, amyotropher Lateralsklerose, primärer Lateralsklerose, Kennedy-Krankheit und Charcot-Marie-Tooth-Erkrankung.

2. Calciumantagonist zur Verwendung gemäß Anspruch 1, **dadurch gekennzeichnet, dass** die motorische Neuropathie infantile spinale Muskelatrophie ist.

3. Calciumantagonist zur Verwendung gemäß Anspruch 1, **dadurch gekennzeichnet, dass** der Calciumantagonist zur Familie der Phenylalkylamine der Formel I oder einer deren Salze gehört, worin:
die Gruppen R₁ und R₂ Parafluorphenylreste sind, und
die Gruppe R₃ ein Phenylrest ist.

4. Calciumantagonist, ausgewählt aus der Gruppe bestehend aus Flunarizin, dessen Salze und Mischungen davon, zur Verwendung in der Behandlung einer motorischen Neuropathie, ausgewählt aus infantiler spinaler Muskelatrophie, amyotropher Lateralsklerose, primärer Lateralsklerose, Kennedy-Krankheit und Charcot-Marie-Tooth-Erkrankung.

5. Calciumantagonist zur Verwendung gemäß Anspruch 4, wobei der Calciumantagonist Flunarizin oder ein Salz davon ist.

6. Calciumantagonist zur Verwendung gemäß Anspruch 4, wobei der Calciumantagonist das Dihydrochlorid von Flunarizin ist.

7. Calciumantagonist zur Verwendung gemäß Anspruch 6, **dadurch gekennzeichnet, dass** der Calciumantagonist in einer Dosis von zwischen 5 mg und 10 mg pro Tag verabreicht wird.

8. Calciumantagonist zur Verwendung gemäß einem der Ansprüche 1 bis 6, **dadurch gekennzeichnet, dass** er die Erhöhung der Verteilung des SMN-Proteins und der snRNPs auf die Cajalkörper ("Corps de Cajal") und/oder die Erhöhung der Zahl der Cajalkörper ermöglicht.

9. Zusammensetzung, umfassend:
einen Calciumantagonisten der Familie der Phenylalkylamine der Formel I und deren Salze, 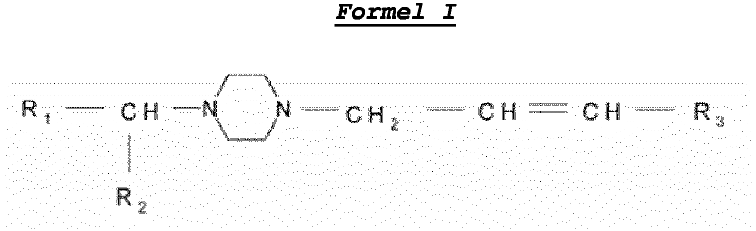 worin:
die Gruppen R₁, R₂ und R₃ identisch oder voneinander verschieden sind und ausgewählt sind aus Phenylresten und Fluorphenylresten, unter der Voraussetzung, dass mindestens eine der Gruppen R₁, R₂ und R₃ ein Fluorphenylrest ist;
und
einen Calciumantagonisten der Familie der Benzofurane der Formel III und deren Salze, 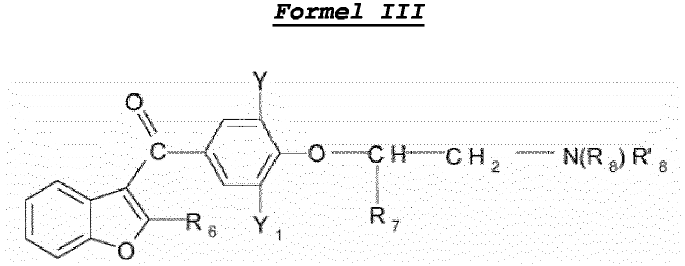 worin:
die Gruppe R₆ für einen niederen Alkylrest mit einer linearen oder verzweigten Kette mit bis zu 6 Kohlenstoffatomen steht,
die Gruppe R₇ für ein Wasserstoffatom oder eine Methylgruppe steht,
die Gruppe N(R₈)R'₈ für eine Dimethylamingruppe, eine Diethylamingruppe, eine Dipropylamingruppe, eine Piperidingruppe, eine Pyrrolidingruppe oder eine Morpholingruppe steht, und
die Gruppen Y und Y₁ identisch sind und für ein Wasserstoffatom, ein Iodatom oder ein Bromatom stehen;
zur Verwendung in der Behandlung einer motorischen Neuropathie, ausgewählt aus infantiler spinaler Muskelatrophie, amyotropher Lateralsklerose, primärer Lateralsklerose, Kennedy-Krankheit und Charcot-Marie-Tooth-Erkrankung.

10. Zusammensetzung zur Verwendung gemäß Anspruch 9, **dadurch gekennzeichnet, dass** der Calciumantagonist der Familie der Phenylalkylamine der Formel I das Dihydrochlorid von Flunarizin ist und, dass der Calciumantagonist der Familie der Benzofurane der Formel III das Hydrochlorid von Amiodaron ist.

11. Zusammensetzung zur Verwendung gemäß Anspruch 9 oder 10, **dadurch gekennzeichnet, dass** sie in einer Dosis von weniger als 5 mg pro Tag verabreicht wird.

## Claims

1. A calcium channel blocker selected from the group consisting of calcium channel blockers of the phenylalkylamine family of formula I and salts thereof, 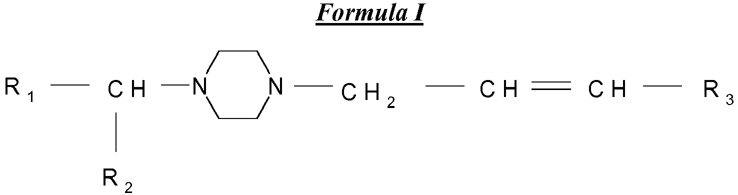 in which :
- the R₁, R₂ and R₃ groups are identical or different and are selected from phenyl and fluorophenyl radicals, provided that at least one of the R₁, R₂ and R₃ groups is a fluorophenyl radical;
for use thereof in the treatment of a motor neuronopathy selected from spinal muscular atrophy, amyotrophic lateral sclerosis, primary lateral sclerosis, Kennedy's disease and Charcot-Marie-Tooth disease.

2. The calcium channel blocker for use thereof as claimed in claim **1**, **characterized in that** said motor neuronopathy is spinal muscular atrophy.

3. The calcium channel blocker for use thereof as claimed in claim **1**, **characterized in that** said calcium channel blocker belongs to the phenylalkylamine family of formula I or a salt thereof,
in which:
- the R₁ and R₂ groups are parafluorophenyl radicals, and
- the R₃ group is a phenyl radical.

4. A calcium channel blocker selected from the group consisting of flunarizine and salts thereof, and mixtures thereof, for use thereof in the treatment of the motor neuronopathy selected from spinal muscular atrophy, amyotrophic lateral sclerosis, primary lateral sclerosis, Kennedy's disease and Charcot-Marie-Tooth disease.

5. The calcium channel blocker for use thereof as claimed in claim **4**, said calcium channel blocker being flunarizine or a salt thereof

6. The calcium channel blocker for use thereof as claimed in claim **4**, said calcium channel blocker being flunarizine dihydrochloride.

7. The calcium channel blocker for use thereof as claimed in claim **6**, **characterized in that** said calcium channel blocker is administered at a dose of between 5 mg and 10 mg per day.

8. The calcium channel blocker for use thereof as claimed in any one of claims **1 to 6**, **characterized in that** it allows an increase in the distribution of the SMN protein and of snRNPs to Cajal bodies and/or an increase in the number of Cajal bodies.

9. A composition comprising:
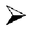 a calcium channel blocker of the phenylalkylamine family of formula I and salts thereof, 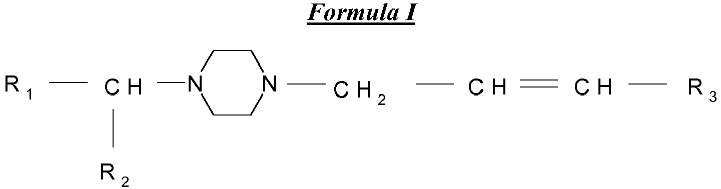 in which :
- the R₁, R₂ and R₃ groups are identical or different and are selected from phenyl and fluorophenyl radicals, provided that at least one of the R₁, R₂ and R₃ groups is a fluorophenyl radical;
and
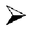 a calcium channel blocker of the benzofuran family of formula III and salts thereof, 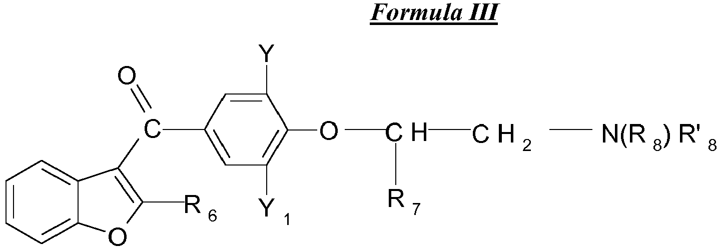 in which :
- the R₆ group represents a linear- or branched-chain lower alkyl radical containing up to six carbon atoms,
- the R₇ group represents a hydrogen atom or a methyl group,
- the N(R₈)R'₈ group represents a dimethylamino, diethylamino, dipropylamino, piperidino, pyrrolidino or morpholino group, and
- the Y and Y₁ groups are identical and represent a hydrogen, iodine or bromine atom;
for use thereof in the treatment of a motor neuronopathy selected from spinal muscular atrophy, amyotrophic lateral sclerosis, primary lateral sclerosis, Kennedy's disease and Charcot-Marie-Tooth disease.

10. The composition for use thereof as claimed in claim **9**, **characterized in that** said calcium channel blocker of the phenylalkylamine family of formula I is flunarizine dihydrochloride and **in that** said calcium channel blocker of the benzofuran family of formula III is amiodarone hydrochloride.

11. The composition for use thereof as claimed in claim **9** or **10**, **characterized in that** it is administered at a dose of less than 5 mg per day.
